# EUROPÄISCHE PATENTANMELDUNG

(11) **EP 1 548 399 A1**
(43) Veröffentlichungstag der Anmeldung: **29.06.2005**
(21) Anmeldenummer: 04106409.8
(22) Anmeldetag: 09.12.2004
(51) Int. Cl.: G01B 11/06, G01N 21/89, G01N 33/34, G01N 23/16, D21G 9/00, G01B 13/22

(54) **Vorrichtung zum Messen einer Eigenschaft einer laufenden Materialbahn**

(30) Priorität: 22.12.2003 DE 10361160
(71) Anmelder: Voith Paper Patent GmbH, 89522 Heidenheim (DE)
(72) Erfinder: Münch, Rudolf, 89551, Königsbronn (DE); Ischdonat, Thomas, 89429, Bachhagel (DE); Typpö, Pekka, ME171NS, Deans Hill, Harrietsham (GB)

(57) **Zusammenfassung**

Eine Vorrichtung zur Messung wenigstens einer Eigenschaft einer Materialbahn, insbesondere Papier- oder Kartonbahn, umfasst auf beiden Bahnseiten vorgesehene bewegliche Messfühler, die unter Bildung eines jeweiligen Luftpolsters mit vorzugsweise zumindest im wesentlichen gleicher Kraft gegen die Bahn drückbar sind, Mittel zum Messen der Luftpolster, insbesondere der Luftpolsterdicke, auf beiden Seiten und wenigstens einen an einem durch ein Luftpolster abgestützten Messfühler angebrachten Eigenschaftssensor, insbesondere Papiereigenschaftssensor.

## Beschreibung

Die Erfindung betrifft eine Vorrichtung zur Messung wenigstens einer Eigenschaft einer Materialbahn, insbesondere Papier- und/oder Kartonbahn.

Es sind bereits verschiedene Papierqualitätssensoren bekannt, bei denen die Messung üblicherweise über einen Messspalt erfolgt, dessen Dicke mehrere Millimeter beträgt. Als für die vorliegende Erfindung relevante Sensoren seien beispielsweise genannt:
Glanzsensor,
Formationssensor,
Oberflächenrauhigkeitssensor,
Flächengewichts- und Aschesensoren (Sensoren mit radioaktiven Quellen),
Aschesensor zur Messung einer jeweiligen Zusammensetzung mittels Röntgenfluoreszenz,
Flächengewichtssensor auf der Basis einer Röntgenröhre zur Messung des Gesamtaschegehalts und der Aschezusammensetzung.

Ein Ziel der Erfindung ist es, eine Familie von mit Luftpolstern versehenen Sensoren zu schaffen, mit der die Probleme im Zusammenhang mit den Messspaltvariationen beseitigt sind. Die meisten Sensoren sind gegenüber solchen Variationen der Spaltdicke empfindlich. Die in der noch nicht veröffentlichten deutschen Patentanmeldung ... (Zeichen der Anmelderin: HEF11948) beschriebene Verwendung stabiler Luftpolster beseitigt die durch Spaltdickenvariationen erzeugten Fehler, während weiterhin ein Kontakt mit dem Papierblatt bzw. der Papierbahn verhindert wird. Dabei werden alle kritischen Komponenten an Messfühlern angebracht, die auf dem Blatt bzw. der Bahn reiten, wobei sie von diesem Blatt bzw. Bahn durch ein Luftpolster getrennt sind, das bezüglich seiner Dicke konstant gehalten ist und eine Dicke besitzt, die lediglich einem Bruchteil eines Millimeters entspricht (üblicherweise 0,1 bis 0,2 mm auf jeder Blattseite).

Die meisten der hier genannten Sensoren sind als solche derzeit zwar erhältlich. Sie erfordern jedoch einen äußerst stabilen und damit kostspieligen Abtastmechanismus, um die Ausrichtung der Sensoren aufrechtzuerhalten, wenn diese über das Blatt bewegt werden. Mit dem weiter unten beschriebenen Luftpolster werden die Anforderungen an die Genauigkeit für den Abtastmechanismus beträchtlich verringert.

Sämtliche Glanzsensoren erfordern eine hochgenaue Ausrichtung zum Blatt, so dass die Anordnung mit einem stabilen Luftpolster eine ideale Plattform für einen Glanzsensor schafft. Dasselbe trifft für einen optischen Formationssensor zur Messung und Analyse der äußerst schnellen Variationen bezüglich der optischen Transmission durch das Blatt zu.

Die Messung der Oberflächenrauhigkeit kann innerhalb des Luftpolsters bzw. Messspaltes entweder durch eine optische Messung der Oberflächentypografie bei hoher Geschwindigkeit oder durch Messung der Luftpolsterdicke, der Luftströmung in das Luftpolster, der Bahngeschwindigkeit und der Betätigungskraft erfolgen. Diese Daten ergeben den Strömungswiderstand, der mit der Oberflächenrauhigkeit korreliert.

Die Flächengewichtsmessung kann innerhalb des Luftpolsters des Messspaltes dadurch erfolgen, dass der Detektor auf einer Blattseite und die radioaktive Betaquelle auf der anderen Blattseite angeordnet bzw. montiert wird. Die radioaktive Quelle erfordert einen Verschlussmechanismus, bei dem es sich beispielsweise um einen gleitenden Verschluss, der sich beim Zurückziehen des Messfühlers über die Luftpolsteroberfläche mit der Quelle bewegt, oder um einen Mechanismus handeln kann, mit dem die Quellenkapsel von der Luftkissenanordnung weg in eine abgeschirmte Position bewegbar ist. Der Vorteil dieser Anordnung besteht darin, dass sowohl die geometrische Empfindlichkeit bezüglich der Messspaltvariation als auch die Fehler eliminiert werden, die durch die variable Luftmasse im Spalt erzeugt werden. Ein weiterer Vorteil ergibt sich daraus, dass mit dem Vorsehen der Quelle und des Detektors die Effizienz bezüglich der Strahlungssammlung wesentlich erhöht wird, womit entweder kleinere Quellen möglich sind oder die Quellen-Lebenszeit verlängert wird. Es erreicht praktisch die gesamte, das Blatt durchdringende Strahlung den Detektor, was eine sehr geringe Ascheempfindlichkeit mit sich bringt. Auch die Empfindlichkeit gegenüber Flattern wird beseitigt.

Mit dieser Vorgehensweise wird auch die Messung von Kaolin Bestandteilen unter Verwendung von Röntgenfluoreszenz erleichtert, solange das Kaolin gleichmäßig in dem Blatt verteilt ist. Mit einem herkömmlichen Sensor würden die geringen, vom Kaolin emittierten Fluoreszenzenergien in der Luft absorbiert werden, ohne den Detektor zu erreichen. Mit einem dünnen und regulierten Luftpolster wird die Absorption in Luft jedoch auf ein Minimum reduziert, was die Messung ermöglicht.

Können alle wesentlichen Aschekomponenten entweder durch Röntgenfluoreszenz oder durch Überwachung der Absorption von Röntgenstrahlung bei verschiedenen Energieniveaus so gemessen werden, dass die Aschekomponenten unabhängig von der Gesamtabsorption bestimmt werden können, dann wird es möglich sein, das Flächengewicht des Blattes mit Röntgenstrahlung zu messen. Ein Röntgensensor (zum Beispiel auf der Basis einer Fe-55-Röhre) kann dann dazu verwendet werden, das Flächengewicht, den Aschegehalt und die Aschezusammensetzung zu messen. Den Schlüssel bildet die Möglichkeit, Kaoline zu messen. TiO₂ und CaCO₃ können selbst bei grössren Messspalten gemessen werden.

Gemäß einem Aspekt schafft die vorliegende Erfindung insbesondere eine Vorrichtung zur Messung wenigstens einer Eigenschaft einer Materialbahn, insbesondere Papier- oder Kartonbahn, mit auf beiden Bahnseiten vorgesehenen bewegl i-chen Messfühlern, die unter Bildung eines jeweiligen Luftpolsters mit vorzugsweise zumindest im Wesentlichen gleicher Kraft gegen die Bahn drückbar sind, mit Mitteln zum Messen der Luftpolster, insbesondere der Luftpolsterdicke, auf beiden Bahnseiten und mit wenigstens einem an einem durch ein Luftpolster gestützten Messfühler angeordneten bzw. angebrachten Eigenschaftssensor, insbesondere Papiereigenschaftssensor.

Ein weiterer Aspekt der Erfindung schafft eine Vorrichtung zur Messung der Papierrauhigkeit, mit auf beiden Papierbahnseiten vorgesehenen beweglichen Messfühlern, die unter Bildung eines jeweiligen Luftpolsters mit vorzugsweise zumindest im Wesentlichen gleicher Kraft gegen die Bahn drückbar sind, stabilen Luftpolstern auf beiden Bahnseiten, Mitteln zur Messung der Luftpolsterdicke, Mitteln zur Messung der Luftströmung in das Luftpolster, Mitteln zur Messung der Luftpolstertemperatur zur Bestimmung der Luftviskosität, Kraftmitteln zum Zusammendrücken des Luftpolsters gegen die Materialbahn, Mitteln zur Berechnung des Strömungswiderstandes basierend auf der Luftpolsterdicke, des Flusses bzw. der Strömung, der Temperatur und der Kraft, und Mitteln zur Berechnung der Oberflächenrauhigkeit basierend auf dem Strömungswiderstand.

Gemäß einem weiteren Aspekt schafft die Erfindung eine Vorrichtung zur Messung wenigstens einer Papiereigenschaft mit auf beiden Papierbahnseiten vorgesehenen beweglichen Messfühlern, die unter Bildung eines jeweiligen Luftpolsters mit vorzugsweise zumindest im Wesentlichen gleicher Kraft gegen die Bahn drückbar sind, stabilen Luftpolstern auf beiden Bahnseiten, einer Strahlungsquelle auf einer Bahnseite und einem Strahlungsdetektor auf der anderen Bahnseite zur Messung von Papiereigenschaften.

Bevorzugte Ausführungsformen der erfindungsgemäßen Messvorrichtung sind in den Unteransprüchen angegeben.

Mit der vorliegenden Erfindung wird eine deutlich höhere Genauigkeit erreicht und ein wesentlich kostengünstigerer Abtastrahmen für verschiedene unterschiedliche Messungen ermöglicht, in dem die Sensoren bzw. Messfühler auf einem stabilen Luftpolster angeordnet sind, wodurch Fehler beseitigt werden, die durch eine Spaltdickenvariation, ein Blattflattern und Luftdichtigkeitsvariationen erzeugt werden.

Die Erfindung wird im Folgenden anhand von Ausführungsbeispielen unter Bezugnahme auf die Zeichnung näher erläutert; in dieser zeigen:
- Fig. 1: eine schematische Darstellung einer Basis-Ausführungsform der Messvorrichtung mit beiderseits der Materialbahn vorgesehenem Glanzsensor,
- Fig. 2: eine schematische Darstellung einer Ausführungsform der Messvorrichtung mit beiderseits der Materialbahn vorgesehenem Glanzsensor mit Referenzstrahl,
- Fig. 3: eine schematische Darstellung einer Ausführungsform der Messvorrichtung, bei der als optischer Papierqualitätssensor ein Formationssensor vorgesehen ist,
- Fig. 4: eine schematische Darstellung einer Ausführungsform der Messvorrichtung, bei der als optischer Papiereigenschaftssensor ein Oberflächenrauhigkeitssensor vorgesehen ist,
- Fig. 5: eine schematische Darstellung einer Ausführungsform der Messvorrichtung mit einem Strahlungssensor als Papiereigenschaftssensor in einer Phase, in der der Strahl bei offenem Verschluss durchgelassen wird,
- Fig. 6: den Strahlungssensor gemäß Fig. 5 bei geschlossenem Verschluss,
- Fig. 7: eine schematische Darstellung einer Messvorrichtung mit einem nuklearen Sensor mit Röntgenfluoreszenz als Papiereigenschaftssensor in einer Phase, in der der Verschluss geöffnet ist, und
- Fig. 8: den nuklearen Sensor gemäß Fig. 7 bei geschlossenem Verschluss.

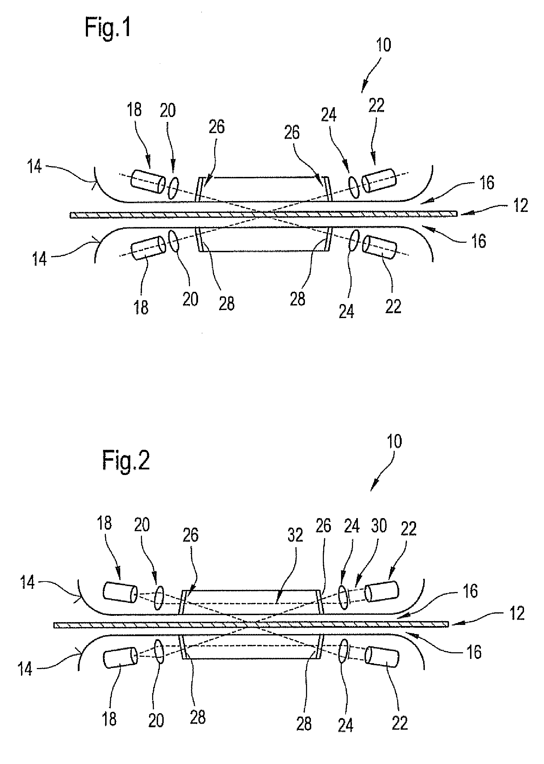

Figur 1 zeigt in schematischer Darstellung eine Basisausführung der Messvorrichtung 10 mit beiderseits des Blattes bzw. der Materialbahn 12, insbesondere Papier- oder Kartonbahn, vorgesehenem Glanzsensor.

Die im vorliegenden Fall beispielsweise zur Messung des Glanzes angeordnete Messvorrichtung 10 umfasst auf beiden Bahnseiten vorgesehene bewegliche Messfühler 14, die unter Bildung eines jeweiligen stabilen Luftpolsters 16 gegen die Materialbahn 12 drückbar sind. Dabei können die Messfühler 14 mit gleicher oder auch mit unterschiedlicher Kraft gegen die Bahn 12 gedrückt werden. Es sind Mittel zum Messen des Luftpolsters, insbesondere der Luftpolsterdicke, auf beiden Bahnseiten vorgesehen. Es ist wenigstens ein an einem Luftpolster 16 abgestützten Messfühler 14 angeordneter bzw. angebrachter Papiereigenschaftssensor vorgesehen. Wie bereits erwähnt, handelt es sich bei dem Papiereigenschaftssensor im vorliegenden Fall um einen beiderseits der Materialbahn 12 angeordneten Glanzsensor.

Die Lichtquelle des Glanzsensors umfasst im vorliegenden Fall zwei weiße LEDs 18 auf den beiden Seiten der Materialbahn 12. Diesen beiden weißen LEDs 18 ist jeweils eine Linse 20 nachgeordnet.

Auf den beiden Seiten der Materialbahn 12 ist überdies jeweils ein Detektor 22 mit vorgeschalteter Linse 24 vorgesehen.

Wie anhand der Figur 1 zu erkennen ist, gelangt der von einer jeweiligen Lichtquelle bzw. LED 18 kommende Lichtstrahl direkt durch das Quellenfenster 26 und das Empfangsfenster 28 zu dem betreffenden Detektor 22 auf der anderen Bahnseite.

Bei dem vorliegenden Glanzsensor handelt es sich also um einen optischen Papiereigenschaftssensor.

Figur 2 zeigt in schematischer Darstellung eine Ausführungsform der Messvorrichtung 10 mit beiderseits der Materialbahn 12 angeordnetem Glanzsensor mit Referenzstrahl. Die vorliegende Ausführungsform unterscheidet sich also von der der Figur 1 im wesentlichen dadurch, dass der von der Lichtquelle bzw. weißen LED 18 kommende Strahl in einen Messstrahl 30 und einen Referenzstrahl 32 aufgeteilt wird, der direkt durch das Quellenfenster 26 und das Empfangsfenster 28 zu einem getrennten Referenzdetektor 22 gelangt. Bei den Detektoren 22 handelt es sich also jeweils um einen so genannten dualen Detektor.

Im übrigen kann die Anordnung gemäß der Figur 2 zumindest im wesentlichen wieder den gleichen Aufbau wie die der Figur 1 besitzen. Einander entsprechenden Teilen sind gleiche Bezugszeichen zugeordnet.

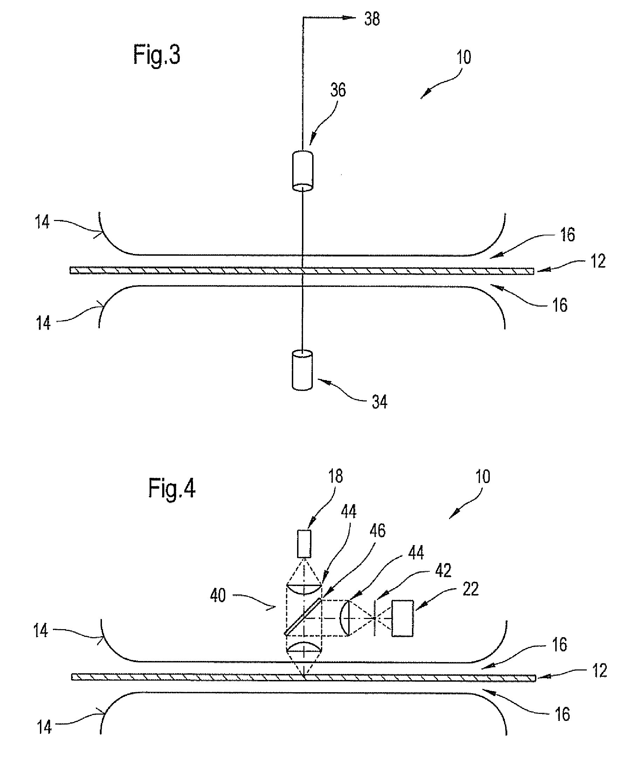

Figur 3 zeigt in schematischer Darstellung eine Ausführungsform der Messvorrichtung 10, bei der als optischer Papiereigenschaftssensor ein Formationssensor vorgesehen ist. Wie anhand der Figur 3 zu erkennen ist, ist auch im vorliegenden Fall zwischen den auf den beiden Bahnseiten angeordneten Messfühlern 14 und der Materialbahn 12 jeweils wieder ein stabiles Luftpolster 16 vorgesehen. Dem unteren Messfühler 14 ist ein Laser 34 und dem oberen Messfühler 14 ein Detektor 36 zugeordnet, dessen Ausgangssignal beispielsweise einem Signalprozessor 38 oder dergleichen zugeführt werden kann.

Figur 4 zeigt in schematischer Darstellung eine Ausführungsform der Messvorrichtung 10, bei der als optischer Papiereigenschaftssensor ein Oberflächenrauhigkeitssensor vorgesehen ist.

Auch im vorliegenden Fall ist zwischen den auf den beiden Bahnseiten angeordneten Messfühlern 14 und der Materialbahn 12 jeweils wieder ein Luftpolster 16 vorgesehen. Die Messvorrichtung 10 bzw. der Oberflächenrauhigkeitssensor kann beispielsweise mit einem Laser bzw. einer LED-Quelle 18 und einer Linsenoptik 40 versehen sein, um die Oberflächentypographie auf der Basis der Messung der Brennfleckgröße auf den Bahnoberfläche zu messen. Dabei kann, wie in Figur 4 dargestellt, vor dem Detektor 22 eine feine Lochblende 42 mit einem Pinhole vorgesehen sein, die bewirkt, dass das vom Detektor 22 gelieferte Signal abnimmt, wenn die Bahnoberfläche sich vom idealen Brennpunkt entfernt.

Wie anhand der Figur 4 zu erkennen ist, umfasst die Linsenoptik 40 außer den Linsen 44 und der feinen Lochblende 42 einen Strahlenteiler 46. Durch diesen Strahlenteiler 46 hindurch gelangt Licht von der Quelle 18 zur Materialbahn 16.

Zudem wird durch diesen Strahlenteiler 46 das von der Materialbahn 12 reflektierte Licht zum Detektor 12 hin umgelenkt.

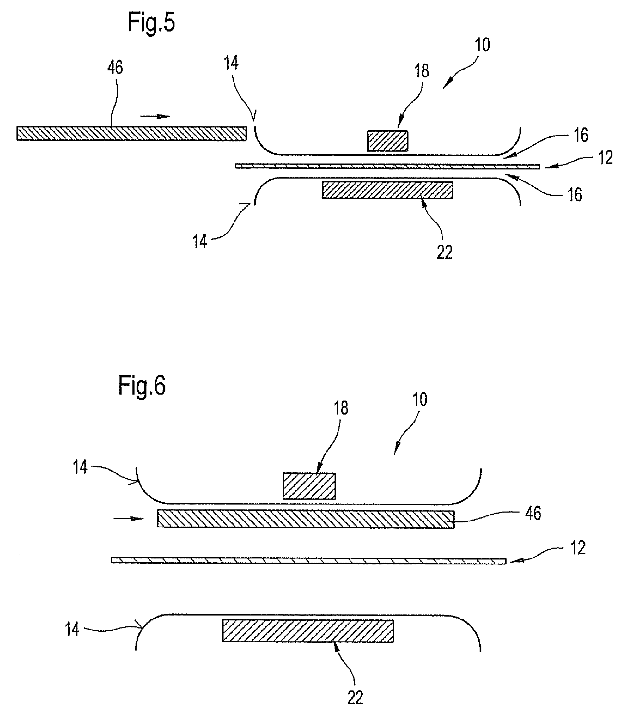

Auch die Figuren 5 und 6 zeigen jeweils wieder eine Messvorrichtung 10 mit auf beiden Papierbahnseiten vorgesehenen beweglichen Messfühlern 14, die unter Bildung eines jeweiligen Luftpolsters 16 mit im wesentlichen gleicher oder unterschiedlicher Kraft gegen die Bahn drückbar sind, stabilen Luftpolstern 16 auf beiden Bahnseiten, einer Strahlungsquelle 18 auf einer Bahnseite und einem Strahlungsdetektor 22 auf der anderen Bahnseite zur Messung einer oder mehrerer Papiereigenschaften. Dabei ist im vorliegenden Fall als Strahlungsquelle 18 eine radioaktive Quelle und als Detektor 22 ein entsprechender Strahlungsdetektor vorgesehen.

Figur 5 zeigt die betreffende Messvorrichtung 10 bzw. den betreffenden Strahlungssensor in einer Phase, in der der Strahl bei offenem Verschluss 48 durchgelassen wird. Figur 6 zeigt den Strahlungssensor gemäß Figur 5 bei geschlossenem Verschluss 46.

Wie anhand der Figuren 5 und 6 zu erkennen ist, ist der Strahl mittels des Verschlusses 46 unterbrechbar, indem dieser über die Lichtquelle 18 geschoben wird, wenn die Messfühler 14 von der Materialbahn 12 zurückgezogen werden.

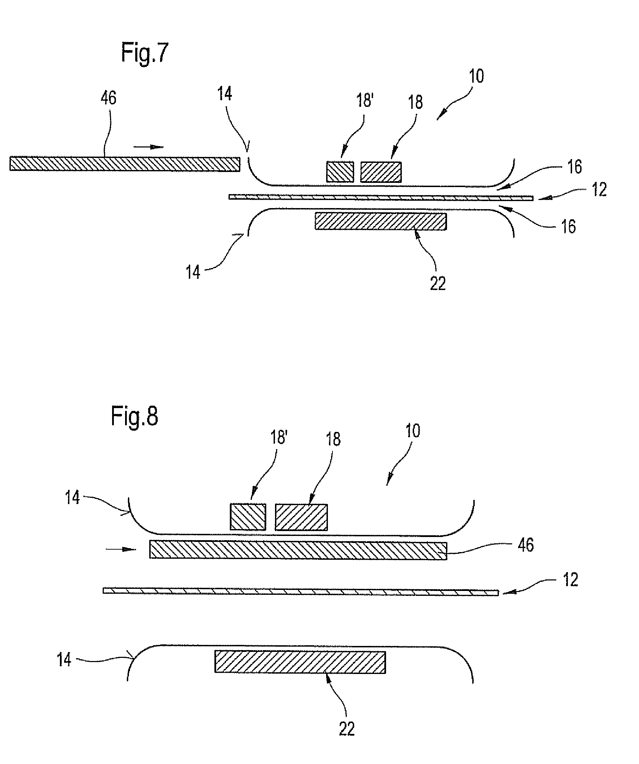

Die Figuren 7 und 8 zeigen eine Ausführungsform der Messvorrichtung 10 mit einem nuklearen Sensor mit Röntgenfluoreszenz. Bei der Quelle 18 handelt er sich im vorliegenden Fall also beispielsweise um eine radioaktive Quelle oder Röntgenröhre und bei dem Detektor 22 auf der gegenüberliegenden Bahnseite um einen entsprechenden Strahlungsdetektor. Auf der gleichen Bahnseite wie die Quelle 18 ist zudem ein Röntgenfluoreszenzdetektor 18' vorgesehen.

Figur 7 zeigt den nuklearen Sensor mit Röntgenfluoreszenz in einer Phase, in der der Verschluss 46 geöffnet ist, d.h. die Strahlung durch die Materialbahn 12 hindurch treten kann.

In Figur 8 ist der nukleare Sensor dagegen wieder in einer Phase gezeigt, in der der Verschluss 46 geschlossen ist.

Wie anhand der Figuren 7 und 8 zu erkennen ist, ist auch im vorliegenden Fall der Verschluss 46 wieder über die Quelle 18 schiebbar, wenn die Messfühler 14 von der Materialbahn 12 zurückgezogen werden.

### Bezugszeichenliste

- 10: Messvorrichtung
- 12: Blatt, Materialbahn
- 14: Messfühler
- 16: Luftpolster
- 18: weiße LED, Lichtquelle, radioaktive Quelle oder Röntgenröhre
- 18': Röntgenfluoreszenzdetektor
- 20: Linse
- 22: Detektor, Referenzdetektor
- 24: Linse
- 26: Quellenfenster
- 28: Empfangsfenster
- 30: Messstrahl
- 32: Referenzstrahl
- 34: Laser
- 36: Detektor
- 38: Signalprozessor
- 40: Linsenoptik
- 42: Lochblende
- 44: Linse
- 46: Strahlenteiler

## Patentansprüche

1. Vorrichtung (10) zur Messung wenigstens einer Eigenschaft einer Materialbahn (12), insbesondere Papier- oder Kartonbahn, mit auf beiden Bahnseiten vorgesehenen beweglichen Messfühlern (14), die unter Bildung eines jeweiligen Luftpolsters (16) mit vorzugsweise zumindest im Wesentlichen gleicher Kraft gegen die Bahn (12) drückbar sind, mit Mitteln zum Messen der Luftpolster (16), insbesondere der Luftpolsterdicke, auf beiden Bahnseiten und mit wenigstens einem an einem durch ein Luftpolster (16) gestützten Messfühler (14) angeordneten bzw. angebrachten Eigenschaftssensor, insbesondere Papiereigenschaftssensor.

2. Messvorrichtung nach Anspruch 1,
**dadurch gekennzeichnet,**
**dass** zumindest ein optischer Eigenschaftssensor vorgesehen ist.

3. Messvorrichtung nach Anspruch 1 oder 2,
**dadurch gekennzeichnet,**
**dass** wenigstens ein insbesondere optischer Glanzsensor vorgesehen ist.

4. Messvorrichtung nach Anspruch 3,
**dadurch gekennzeichnet,**
**dass** ein beiderseits der Materialbahn (12) angeordneter Glanzsensor vorgesehen ist.

5. Messvorrichtung nach Anspruch 3 oder 4,
**dadurch gekennzeichnet,**
**dass** der von der Lichtquelle (18) kommende Strahl in einen Messstrahl (30) und einen Referenzstrahl (32) aufgeteilt wird, der direkt durch das Quellenfenster (26) und das Empfangsfenster (28) zu einem getrennten Referenzdetektor (12) gelangt.

6. Messvorrichtung nach Anspruch 5,
**dadurch gekennzeichnet,**
**dass** der Glanzsensor beiderseits der Materialbahn (12) angeordnet ist.

7. Messvorrichtung nach einem der vorhergehenden Ansprüche,
**dadurch gekennzeichnet,**
**dass** die Lichtquelle eines jeweiligen Glanzsensors eine insbesondere weiße LED (Licht emittierende Diode) (18) umfasst.

8. Messvorrichtung nach einem der vorhergehenden Ansprüche,
**dadurch gekennzeichnet,**
**dass** wenigstens ein insbesondere optischer Formationssensor vorgesehen ist.

9. Messvorrichtung nach einem der vorhergehenden Ansprüche,
**dadurch gekennzeichnet,**
**dass** wenigstens ein insbesondere optischer Eigenschaftssensor zur Messung der Oberflächenrauhigkeit der Bahn (12) vorgesehen ist.

10. Messvorrichtung nach Anspruch 9,
**dadurch gekennzeichnet,**
**dass** wenigstens ein optischer Oberflächenrauhigkeitssensor mit einer LED-Quelle (18) und einer Linsenoptik (40) vorgesehen ist, um die Oberflächentypografie auf der Basis der Messung der Brennfleckgröße auf der Bahnoberfläche zu messen (insbesondere mit einer Geometrie entsprechend der Darstellung in Figur 3).

11. Messvorrichtung nach Anspruch 10,
**dadurch gekennzeichnet,**
**dass** vor dem Detektor (22) eine feine Lochblende (42) mit einem Pinhole vorgesehen ist, die bewirkt, dass das vom Detektor (22) gelieferte Signal abnimmt, wenn die Bahnoberfläche sich vom idealen Brennpunkt entfernt.

12. Vorrichtung (10) zur Messung der Papierrauhigkeit, mit auf beiden Papierbahnseiten vorgesehenen beweglichen Messfühlern (14), die unter Bildung eines jeweiligen Luftpolsters (16) mit vorzugsweise zumindest im Wesentlichen gleicher Kraft gegen die Bahn (12) drückbar sind, stabilen Luftpolstern (16) auf beiden Bahnseiten, Mitteln zur Messung der Luftpolsterdicke, Mitteln zur Messung der Luftströmung in das Luftpolster (16), Mitteln zur Messung der Luftpolstertemperatur zur Bestimmung der Luftviskosität, Kraftmitteln zum Zusammendrücken des Luftpolsters (16) gegen die Materialbahn (12), Mitteln zur Berechnung des Strömungswiderstandes basierend auf der Luftpolsterdicke, des Flusses bzw. der Strömung, der Temperatur und der Kraft, und Mitteln zur Berechnung der Oberflächenrauhigkeit basierend auf dem Strömungswiderstand.

13. Vorrichtung (10) zur Messung wenigstens einer Papiereigenschaft mit auf beiden Papierbahnseiten vorgesehenen beweglichen Messfühlern (14), die unter Bildung eines jeweiligen Luftpolsters (16) mit vorzugsweise zumindest im Wesentlichen gleicher Kraft gegen die Bahn (12) drückbar sind, stabilen Luftpolstern (16) auf beiden Bahnseiten, einer Strahlungsquelle (18) auf einer Bahnseite und einem Strahlungsdetektor (22) auf der anderen Bahnseite zur Messung von Papiereigenschaften.

14. Messvorrichtung nach Anspruch 13,
**dadurch gekennzeichnet,**
**dass** die Strahlungsquelle (18) eine Betaquelle wie insbesondere eine Kr-85- oder Pm-147-Quelle für eine Flächengewichtsmessung umfasst.

15. Messvorrichtung nach Anspruch 13,
**dadurch gekennzeichnet,**
**dass** die Strahlungsquelle (18) eine Röntgenstrahlungsquelle wie insbesondere eine Fe-55-Quelle oder Röntgenröhre zur Messung des Papieraschegehalts umfasst.

16. Messvorrichtung nach einem der vorhergehenden Ansprüche,
**dadurch gekennzeichnet,**
**dass** der Strahl mittels eines Verschlusses (46) unterbrechbar ist, der über die Quelle (18) schiebbar ist, wenn die Messfühler (14) zurückgezogen werden.

17. Messvorrichtung nach einem der vorhergehenden Ansprüche,
**dadurch gekennzeichnet,**
**dass** der Strahl dadurch unterbrechbar ist, dass die Quelle (18) von dem Messfühler (14) weg in eine abgeschirmte Position bewegt wird, wenn die Messfühler (14) von der Papierbahn (12) zurückgezogen werden.

18. Messvorrichtung nach einem der vorhergehenden Ansprüche,
**dadurch gekennzeichnet,**
**dass** ein Röntgenfluoreszenzdetektor (18') vorgesehen ist, um die Blatt- bzw. Bahnaschegehaltzusammensetzung zu messen.

19. Messvorrichtung nach Anspruch 18,
**dadurch gekennzeichnet,**
**dass** die Röntgenfluoreszenzeinrichtung zur Messung der Aschegehaltzusammensetzung auch zur Tonmessung vorgesehen ist.

20. Messvorrichtung nach einem der vorhergehenden Ansprüche,
**dadurch gekennzeichnet,**
**dass** sie eine Röntgenröhre und mehrere Röntgenfilter umfasst, die der Reihe nach in den Strahl einführbar sind, um die Röntgenstrahlentransmission bei verschiedenen Röntgenstrahlungsspektren zu messen und die Aschezusammensetzung und/oder den Gesamtaschegehalt des Blattes bzw. der Bahn (12) zu bestimmen.

21. Messvorrichtung nach Anspruch 19 oder 20,
**dadurch gekennzeichnet,**
**dass** Mittel vorgesehen sind, um unter Verwendung der Daten bezüglich der Aschezusammensetzung zusammen mit der Gesamtröntgenstrahlungsabsorption das Flächengewicht zu messen.

22. Messvorrichtung nach einem der vorhergehenden Ansprüche,
**dadurch gekennzeichnet,**
**dass** zumindest eines der Luftpolster (16) eine größere Dicke und insbesondere eine Dicke im Bereich von mehreren Millimetern besitzt.

23. Messvorrichtung nach einem der vorhergehenden Ansprüche,
**dadurch gekennzeichnet,**
**dass** die Kräfte, mit denen die beiderseits der Papierbahn (12) vorgesehenen Luftpolster (16) auf das Papier wirken, ungleich sind.

24. Messvorrichtung nach einem der vorhergehenden Ansprüche,
**dadurch gekennzeichnet,**
**dass** nur eine Seite des Sensors unter Bildung wenigstens eines Luftpolsters (16) gegen das Blatt bzw. die Bahn (12) gedrückt ist und dass die andere Seite entweder schwimmt oder in festem Kontakt mit einer Platte oder Walze steht, insbesondere für eine einseitige Messung.

25. Messvorrichtung nach einem der vorhergehenden Ansprüche,
**dadurch gekennzeichnet,**
**dass** die Dicke des Gasamtspaltes zwischen den Messfühlern (14) durch Rückkopplung von den gesamten Druckluftmessungen und/oder entsprechenden Abstands- oder Dickenmessungen konstant gehalten ist.
